# NEW EUROPEAN PATENT SPECIFICATION

(11) **EP 1 589 876 B2**
(45) Date of publication and mention of the opposition decision: **17.02.2021**
(45) Mention of the grant of the patent: 19.10.2011
(21) Application number: 04703444.2
(22) Date of filing: 20.01.2004
(51) Int. Cl.: A61B 6/03, A61B 5/113, A61N 5/10

(54) **COMPUTED TOMOGRAPHY SCANNING**
COMPUTERTOMOGRAPHISCHE UNTERSUCHUNG
BALAYAGE TOMOGRAPHIQUE PAR ORDINATEUR

(30) Priority: 21.01.2003 GB 0301278
(43) Date of publication of application: 02.11.2005
(73) Proprietor: Elekta AB (publ), 103 93 Stockholm (SE)
(72) Inventor: BROWN, Kevin, John, Horsham, West Sussex RH13 5EJ (GB); JAFFRAY, David, Etobicoke, Ontario M9A4Z3 (CA); SIEWERDSEN, Jeffrey, Toronto, Ontario M5J2Y7 (CA); VAN HERK, Marcel, NL-1064 DE Amsterdam (NL); SONKE, Jan-Jakob, NL-1016 VV Amsterdam (NL)
(74) Representative: Downing, Michael Philip
(86) International application number: PCT/GB2004/000220
(87) International publication number: WO 2004/064641

(56) References cited:
- EP-A1- 1 061 474
- EP-A1- 1 088 517
- EP-A1- 1 244 058
- EP-A2- 1 321 100
- WO-A-01/67960
- WO-A1-03/003796
- WO-A1-2004/054443
- WO-A1-2004/064641
- WO-A2-01/60236
- WO-A2-01/67960
- WO-A2-02/26125
- WO-A2-2004/010383
- US-A- 5 137 026
- US-A- 5 254 948
- US-A- 5 287 276
- US-A1- 2001 048 731
- US-A1- 2002 025 017
- US-A1- 2002 131 545
- US-A1- 2003 007 593
- US-A1- 2003 007 593
- US-A1- 2003 007 601
- US-A1- 2003 007 601
- US-A1- 2003 015 207
- US-A1- 2003 016 782
- US-A1- 2003 016 851
- US-A1- 2005 169 420
- US-B1- 6 385 288
- PATENT ABSTRACTS OF JAPAN vol. 2000, no. 10, 17 November 2000 (2000-11-17) -& JP 2000 201920 A (FUJI PHOTO FILM CO LTD), 25 July 2000 (2000-07-25)
- Kachelriess, M. et al: "Kymogram detection and kymogram-correlated image reconstruction from subsecond spiral computed tomography scans of the heart", Med. Phys., vol. 29, no. 7, July 2002 (2002-07), pages 1489-1503, & Kachelriess, M. et al: "ECG-correlated image reconstruction from subsecond multi-slice spiral CT scans of the heart", Med. Phys., vol. 27, no. 8, August 2000 (2000-08), pages 1881-1902,
- Kachelriess, M., Kalender, W.: "Electrocardiogram-correlated image reconstruction from subsecond spiral computed tomography scans of the heart", Med. Phys., vol. 25, no. 12, December 1998 (1998-12), pages 2417-2431,
- Dhanantwari, A.C. et al: "Correcting organ motion artifacts in x-ray CT systems based on tracking of moton phase by the spatial overlap correlator. II. Experimental study", Med. Phys., vol. 28, no. 8, August 2001 (2001-08), pages 1577-1596,
- Nelson, T.R.and Pretorius, H.: "Three-dimensional ultrasound imaging", Ultrasound in Med. & Biol, vol. 24, no. 9, 1998, pages 1243-1270,
- Nelson, T.R: "Three-dimensional fetal echocardiography", Progress in Biophysics & Molecular Biology, vol. 69, 1998, pages 257-272,
- Manzkce, R. et al: "Artifact Analysis and Reconstruction improvement in Helical Cardiac Cone Beam CT", IEEE Transactions on medical imaging, vol. 23, no. 9, September 2004 (2004-09), pages 1150-1164,
- Grass, M. et al: "Helical cardiac cone beam reconstruction using retrospective ECG gating", Phys. Med. Biol, vol. 48, 2003, pages 3069-3084,
- Rosenzweig et al: "The Deep Inspiration Breath-Hold Technique In the Treatment of Inoperable Non-Small-Cell Lung Cancer", Int J. Radiation Oncology Biol. Phys, vol. 48, no. 1, 2000, pages 81-87,
- Hutchinson, John: "On the Capacity of the Lungs, and on the Respiratory Functions", , 1846, pages 1, 98-103,

## Description

### FIELD OF THE INVENTION

The present invention relates to scanning by computed tomography (CT).

### BACKGROUND ART

CT scanning is a process for imaging the internal structure of a patient. In conventional CT scanning, a beam of x-rays is projected through the patient and its attenuation is measured. At the same time, the apparatus is rotated about an axis passing longitudinally through the patient. Thus, data is acquired as to the attenuation of the beam in each direction in the plane in which rotation takes place. From this data, the internal structure of the patient on that plane can be computed. The patient or apparatus is then indexed along the axis and a further plane (known as a 'slice') is then investigated. A three dimensional image of the patient can then be constructed from the various slices.

One problem is that over the time required to acquire the necessary slices, the patient is not motionless. Gross motor movement can be avoided by suitable instruction to the patient, but even so each slice is acquired at a different phase of the breathing cycle. This results in a beating artefact due to the different frequency of breathing and slice acquisition.

Two ways have been used to solve this problem. On is to trigger the CT on a particular phase of the patients breathing. This is termed 'respiration gated CT' and implies that one CT slice is acquired for every breath. This means that it takes a long time to acquire a complete volume of data.

Another technique is to monitor the phase of the patients breathing whilst acquiring CT slices continuously. Once the data is acquired, slices that have comparable breathing phase are selected from the complete set and these are then used to visualise the volume. This has the advantage that any phase can be selected retrospectively and therefore the effect of breathing can be studied. This is termed 'respiration correlated CT'.

Conventional CT scans have the disadvantage that the resolution along the axis is poor since it corresponds to the slice thickness. It is theoretically straightforward to increase this, but doing so results in a correspondingly longer acquisition time, or the need to rotate the apparatus correspondingly faster. Both options also give rise to an attendant reduction in contrast in the measured beam. Accordingly, 'cone beam CT' methods have been developed, in which a conical beam of radiation is directed at the patient and a two-dimensional image acquired via a flat panel detector. This apparatus is then rotated around the patient axis and a three-dimensional image is reconstructed from the set of two-dimensional images. As the individual slices are eliminated, there is the same resolution in all directions of the image. Likewise, as there are no slices the above-mentioned breathing artefact is absent since there can be no variation in patient position between slices.

US-A-2003/0007601 discloses a radiation therapy system comprising: a radiation source that moves about a path and directs a beam of radiation towards an object; a cone-beam computer tomography system comprising: an x-ray source that emits an x-ray beam in a cone-beam form towards said object; an amorphous silicon flat-panel imager receiving x-rays after they pass through the object, said imager providing an image of said object; and a computer connected to said radiation source and said cone beam computerized tomography system, wherein said computer receives said image of said object and based on said image sends a signal to said radiation source that controls said path of said radiation source. The timing of x-ray exposures, gantry rotation, and flat panel imager readout may be synchronized by an external trigger mechanism (gating source), such as a device for active breathing control, whereby the gating source triggers x-ray production, gantry rotation, and flat panel imager readout in a manner synchronized with the motion of anatomical structures in the patient in order to reduce the deleterious effects of organ motion in image reconstructions.

### SUMMARY OF THE INVENTION

We have however found that there are other artefacts in the reconstructed volume data of cone beam CT systems, which we have traced to patient breathing movements. In addition, the motion is not measurable in the reconstructed volume data. This can be a particular problem in cone beam systems due to the long time required for acquisition, typically 1-2 minutes.

The techniques used in conventional CT scanners cannot be used directly in a cone beam system as the data is acquired in 2D projection images, and therefore slices cannot be selected from the resulting data. However, respiration correlation techniques could be applied to the acquired projection images rather than the reconstructed CT volume. To achieve this, we propose monitoring the phase of the patients breathing while acquiring projection images continuously. On completion of the acquisition, projection images that have comparable breathing phases can be selected from the complete set, and these are used to reconstruct the volume data using similar techniques to those of conventional CT. An advantage is that any phase can be selected and therefore the effect of breathing can be studied.

Breath control systems are available, intended for use in conventional CT scanning, and which could be used to monitor the patient's breathing. According to the invention, however, a feature in the projection image(s) is used to determine the breathing phase. A suitable feature is the position of the patient's diaphragm. This can then be used to select the relevant images to be used in the projection process.

It is known in the field of conventional CT scanning to be advantageous to prompt the patient visually and audibly in order to ensure a regular amplitude and pattern of breathing. Techniques such as these could usefully be applied in the present invention.

Furthermore this feature in the projection images can be used to control delivery of therapeutic radiation dependent on the patient's breathing cycle, to ensure that the tumour is in the correct position when the radiation is delivered. This will provide a direct measure of the patient's breathing phase, a significant improvement as compared to current methods that use external markers affixed to the patient. The use of a 3D volume data set generated using the same patient position and contemporaneous with the treatment will remove significant uncertainties.

The present invention further provides a radiotherapy device as defined in claim 6, comprising a respiration correlated cone beam CT scanner and a source of therapeutic radiation, in which therapeutic radiation is delivered during the scan at times correlated with the patient's breathing cycle.

### BRIEF DESCRIPTION OF THE DRAWINGS

An embodiment of the present invention will now be described by way of example, with reference to the accompanying figures in which;
Figure 1 is a view of a cone beam CT scanner according to the present invention, viewed along the axis of rotation thereof;
Figure 2 is a schematic view of the system incorporating such a scanner; and
Figure 3 shows a treatment apparatus including the scanner of the present invention.

### DETAILED DESCRIPTION OF THE EMBODIMENT

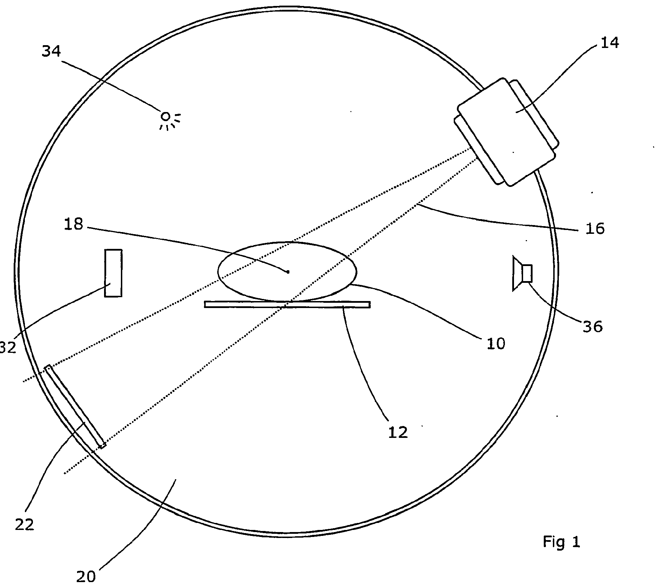

Figure 1 shows a cone beam CT scanner. A patient 10 is supported on a couch 12 which may be of any suitable design. Couches typically allow the elevation and longitudinal position of the patient to be adjusted and this may be provided for as desired.

An x-ray source 14 is arranged to project a wide beam 16 of radiation generally directed towards the isocentre 18 of the patient. The source 14 is rotatable around the isocentre 18 on a rotational support 20. The support can, for example, be in the form of a ring or annulus around the patient 10 and couch 12 in which the source is mounted, or it can be a C-arm, or any suitable support allowing the source to rotate, or any combination thereof.

A two-dimensional flat-panel detector 22 is also mounted on the support 20, opposite the source 14 and arranged to rotate in synchronism therewith. If the support includes a C-arm then this can be achieved by mounting the detector on the opposite arm.

Thus, radiation emitted by the source 14 is partially absorbed by the patient and the attenuated signal is detected by the flat panel detector 22. The source 14 and detector 22 are then indexed rotationally and a fresh image obtained. This is repeated until sufficient images are acquired to reconstruct the volume data, typically one complete rotation.

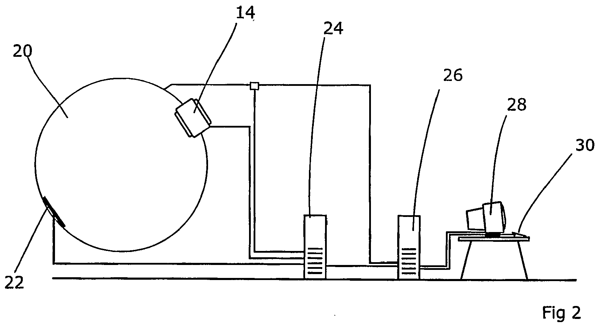

Figure 2 shows the system as a whole. The scanner of figure 1 is shown, together with cables linking the source 14, detector 22 and rotational support 20 to a plurality of computing means 24, 26 which process the data generated including the images, source intensity (etc), and rotational support position. Data is output via any suitable means, depicted generally as a monitor 28 but not limited thereto, and the system is controlled by any suitable input means, again depicted generally as a keyboard 30 but likewise not especially limited thereto.

As mentioned above, we have found that there are artefacts in the reconstructed volume data of cone beam CT systems, which we have traced to patient breathing movements. To overcome or alleviate these, respiration correlation techniques are applied to the acquired projection images by the computing means 24, 26. This differs from conventional respiration-correlated CT scanning in acting on the acquired projection images rather than the reconstructed CT volume.

To assist in this process, a breath control system is provided at 32 to monitor the phase of the patients breathing while the projection images are acquired. On completion of the acquisition, projection images that have comparable breathing phases can be selected from the complete set, and these are used to reconstruct the volume data using cone beam CT techniques. As a result, any phase or range of phases can be selected and therefore the effect of breathing can be studied if desired.

According to the invention a feature in the projection image(s) is used to determine the breathing phase, such as the position of the patient's diaphragm. This can then be used to select the relevant images to be used in the projection process.

An alert system including a light 34 and a buzzer 36 is provided, to prompt the patient visually and audibly in order to ensure a regular amplitude and pattern of breathing. Other alerts could of course be employed, such as other forms of visible prompts including (for example) movable devices, and other forms of audible prompts including (for example) speakers, percussive devices or any other form of controllable sound generation apparatus.

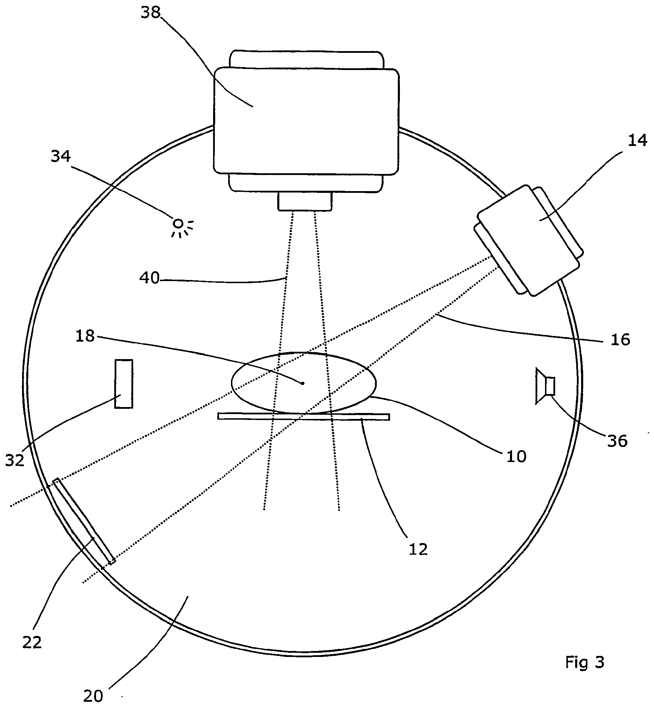

Figure 3 shows a system including a therapeutic source of radiation 38 arranged to emit a suitably collimated beam of therapeutic radiation 40. This allows simultaneous scanning and treatment. If the radiation from source 14 continues during the treatment the selected feature (above) in the projection images can be used to control delivery of therapeutic radiation from the source 38, dependent on the patient's breathing cycle. This ensures that the tumour is in the correct position when the radiation is delivered. This will provide a direct measure of the patient's breathing phase, a significant improvement as compared to current methods that use external markers affixed to the patient. The use of the same direct measure of the patient's breathing phase and patient position to generate the 3D volume data set and control the treatment delivery will remove significant uncertainties.

It will of course be understood that many variations may be made to the above-described embodiment without departing from the scope of the present invention.

## Claims

1. A cone beam CT scanner acquiring two-dimensional projection images and including means for acquiring information as to the patient respiration cycle from a feature in the projection image to determine the breathing phase and means for selection of acquired two-dimensional projection Images from the set of data acquired during a scan on the basis of the acquired respiration cycle information.

2. A cone beam CT scanner according to claim 1, adapted to monitor the phase of the patient's breathing, via the means for acquiring information, continuously during acquisition of projection images.

3. A cone beam CT scanner according to claim 2 arranged to select from the complete data set the projection images with comparable breathing phases on completion of the acquisition process.

4. A cone beam CT scanner according to claim 1 in which the feature in the projection image(s) is the position of the patient's diaphragm.

5. A cone beam CT scanner according to any one of the preceding claims including means to provide visual and/or audible prompts for the patient's breathing.

6. A radiotherapy device comprising a cone beam CT scanner and a source of therapeutic radiation, in which the CT scanner is adapted to apply respiration correlation techniques to the acquired two-dimensional projection images and the source is adapted to deliver therapeutic radiation during the scan at times correlated with the patient's breathing cycle.

## Patentansprüche

1. Kegelstrahlcomputertomograph zur Erstellung von zweidimensionalen Projektionsbildern einschließlich Mitteln zur Erfassung von Informationen über den Atmungszyklus des Patienten über eine Funktion in dem/den Projektionsbild(ern) zum Bestimmen der Atmungsphase sowie Mitteln zur Auswahl von erstellten zweidimensionalen Projektionsbildern aus dem Datensatz, der während eines Scans aufgenommen wurde, basierend auf den Informationen, die über den Atmungszyklus erworben wurden.

2. Kegelstrahlcomputertomograph nach Anspruch 1, der zur Überwachung der Atmungsphasen des Patienten über die Mittel zur Erfassung von Informationen kontinuierlich während der gesamten Erstellung der Projektionsbilder eingerichtet ist.

3. Kegelstrahlcomputertomograph nach Anspruch 2, der so gestaltet ist, dass nach Abschluss des Erfassungsprozesses die Projektionsbilder mit vergleichbaren Atmungsphasen aus dem vollständigen Datensatz ausgewählt werden.

4. Kegelstrahlcomputertomograph nach Anspruch 1, wobei das Merkmal in dem/den Projektionsbild(ern) die Position des Zwerchfells des Patienten ist.

5. Kegelstrahlcomputertomograph nach einem der vorangehenden Ansprüche, einschließlich Mitteln zur Bereitstellung von visuellen und/oder akustischen Abfragen über die Atmung des Patienten.

6. Strahlentherapievorrichtung, die einen Kegelstrahlcomputertomographen und eine Quelle für therapeutische Strahlung umfasst, wobei der Computertomograph dazu eingerichtet ist, Atmungskorrelationstechniken auf die erfassten zweidimensionalen Projektionsbilder anzuwenden, und die Quelle dazu eingerichtet ist, die therapeutische Strahlung während des Scans zu Zeiten bereitzustellen, die mit dem Atmungszyklus des Patienten korreliert sind.

## Revendications

1. Tomodensitomètre à faisceau conique acquérant des images de projection bidimensionnelles, et comprenant un moyen permettant d'acquérir des informations sur le cycle de respiration du patient à partir d'une caractéristique dans la ou les images de projection pour déterminer la phase de respiration et des moyens permettant de sélectionner des images de projection bidimensionnelles acquises à partir du jeu de données acquis au cours d'un scannage, en fonction des informations acquises sur le cycle de respiration.

2. Tomodensitomètre à faisceau conique conforme à la revendication 1, adapté pour contrôler, via les moyens permettant d'acquérir des informations, la phase de respiration du patient en continu au cours de l'acquisition des images de projection.

3. Tomodensitomètre à faisceau conique conforme à la revendication 2, conçu pour sélectionner, à partir du jeu de données complet, les images de projection avec des phases de respiration comparables, à l'achèvement du processus d'acquisition.

4. Tomodensitomètre à faisceau conique conforme à la revendication 1, dans lequel la caractéristique dans la ou les images de projection est la position du diaphragme du patient.

5. Tomodensitomètre à faisceau conique conforme à une quelconque des revendications précédentes, comportant un moyen permettant de fournir des messages d'invite visuels et/ou audibles pour la respiration du patient.

6. Dispositif de radiothérapie comprenant un tomodensitomètre à faisceau conique et une source de radiations thérapeutiques, dans lequel le tomodensitomètre est adapté pour appliquer des techniques de corrélation de la respiration aux images de projection bidimensionnelles acquises et la source est adaptée pour produire un rayonnement thérapeutique au cours du scannage, dans des périodes en corrélation avec le cycle de respiration du patient.
